# EUROPEAN PATENT APPLICATION

(11) **EP 4 534 697 A1**
(43) Date of publication of application: **09.04.2025**
(21) Application number: 23812167.7
(22) Date of filing: 25.05.2023
(51) Int. Cl.: C12Q 1/6886

(54) **METHOD FOR DETECTING LUNG CANCER**

(30) Priority: 27.05.2022 KR 20220065224
(71) Applicant: Genomictree, Inc., Daejeon 34027 (KR)
(72) Inventor: AN, Sungwhan, Daejeon 34125 (KR); OH, Taejeong, Daejeon 34127 (KR)
(74) Representative: Mewburn Ellis LLP
(86) International application number: PCT/KR2023/007168
(87) International publication number: WO 2023/229393

(57) **Abstract**

The present invention relates to a method for providing information for lung cancer diagnosis, a composition for diagnosing lung cancer, and a kit comprising same and, more specifically, to a method for providing information for lung cancer diagnosis by using a primer for specifically amplifying a plurality of methylated lung cancer marker genes, a composition for diagnosing lung cancer, and a kit comprising same.

## Description

### Technical Field

The present invention relates to a method for providing information for lung cancer diagnosis, a composition for diagnosing lung cancer, and a kit comprising same and, more specifically, to a method for providing information for lung cancer diagnosis by using a primer for specifically amplifying a plurality of methylated lung cancer marker genes, a composition for diagnosing lung cancer, and a kit comprising same.

### Related Art

In the genomic DNA of mammalian cells, in addition to A, C, G, and T, there is a fifth base, 5-methylcytosine (5-mC) in which a methyl group is attached to the fifth carbon of the cytosine ring. The 5-mC is always at the C of the CG dinucleotide only (5'-mCG-3'), and this CG is often labeled CpG. The C of CpG is mostly methylated with a methyl group attached. Methylation of this CpG inhibits the expression of repetitive sequence in the genome, such as alu and transposon, and is the most common site of extragenomic changes in mammalian cells. The 5-mC of this CpG is spontaneously deaminated and changed to T, resulting in CpG in the mammalian genome appearing only 1 % of the time, far less than it should normally (1/4 x 1/4 = 6.25%).

Some CpGs appear exceptionally densely clustered, and these are called CpG sites (CpG islands). CpG sites are defined as regions that are 0.2-3 kb in length, have C and G base distribution percentage of more than 50%, and a high concentration of CpGs with a distribution percentage of at least 3.75%. There are about 45,000 CpG sites in the entire human genome, and they are particularly concentrated in promoter regions that regulate gene expression. In fact, CpG sites appear in the promoters of housekeeping genes, which account for about half of all human genes (Cross, S. et al., Curr. Opin. Gene Develop., 5:309, 1995). Aberrant DNA methylation is known to occur primarily in the 5' regulatory region of a gene, resulting in reduced expression of that gene.

In normal human somatic cells, the CpG islands in the promoter regions of the housekeeping genes are not methylated, but genes that are imprinted not to be expressed during development and genes on the X chromosome that are inactivated are methylated.

During carcinogenesis, promoter CpG islands are methylated and the expression of the corresponding genes is disturbed. In particular, methylation of CpG islands in the regulatory regions of tumor suppressing genes that regulate cell cycle and apoptosis, repair DNA, get involved in cell adhesion and interaction between cells, and inhibit invasion and metastasis, blocks the expression and function of these genes, as in a mutation in the coding sequence, and accordingly promotes the development and progression of cancer. In addition, partial methylation of CpG islands occurs with aging.

Methylation of regulatory region of tumor-related genes is an important marker of cancer, which can be used in a variety of ways, including diagnosis and early detection of cancer, prediction of carcinogenic risk, prognosis of cancer, post-treatment follow-up, and prediction of response to chemotherapy. In fact, there have been recent attempts to investigate the promoter methylation of tumor-related genes in blood, sputum, saliva, feces, urine, etc. and use it for various cancer therapies (Ahlquist, D.A. et al., Gastroenterol., 119:1219, 2000).

Under this technical background, the inventors of the present invention have found that methylation of lung cancer marker genes can be detected with high detection sensitivity and accuracy by using a primer that specifically amplifies a plurality of methylated lung cancer marker genes, and have completed the present invention.

### Summary of Invention

An object of the present invention is to provide a method for providing information for lung cancer diagnosis by using a primer that specifically amplifies a plurality of methylated lung cancer marker genes.

It is an object of the present invention to provide a method for diagnosing lung cancer by using a primer that specifically amplifies a plurality of methylated lung cancer marker genes.

An object of the present invention is to provide a composition for diagnosing lung cancer by using a primer that specifically amplifies a plurality of methylated lung cancer marker genes.

It is an object of the present invention to provide a kit for diagnosing lung cancer comprising the composition.

To achieve the above objectives, the present invention relates to a method for providing information for lung cancer diagnosis, comprising the following steps: (a) treating a sample with one or more reagents that differentially modify the methylated PCDHGA12 (Protocadherin Gamma Subfamily A, 12) gene and the CDO1 (Cysteine Dioxygenase Type 1) gene, the unmethylated PCDHGA12 gene and the CDO1 gene; and (b) treating a primer that specifically amplifies the methylated PCDHGA12 gene and the CDO1 gene.

The present invention also provides a composition for diagnosing lung cancer, comprising (a) one or more reagents that differentially modify the methylated PCDHGA12 (Protocadherin Gamma Subfamily A, 12) gene and the CDO1 (Cysteine Dioxygenase Type 1) gene, the unmethylated PCDHGA12 gene and the CDO1 gene; and (b) a primer that specifically amplifies the methylated PCDHGA12 gene and the CDO1 gene.

The present invention also provides a kit for diagnosing lung cancer comprising the composition.

### Brief Description of Drawing

FIG. 1 shows the results of methylation-specific amplification of PCDHGA12 and CDO1 genes by methylation-specific primers.
FIG. 2 shows the methylation status of PCDHGA12 and CDO1 genes in bronchial lavage fluid from patients with benign lung disease and lung cancer, and the results of receiver operating characteristic (ROC) analysis.

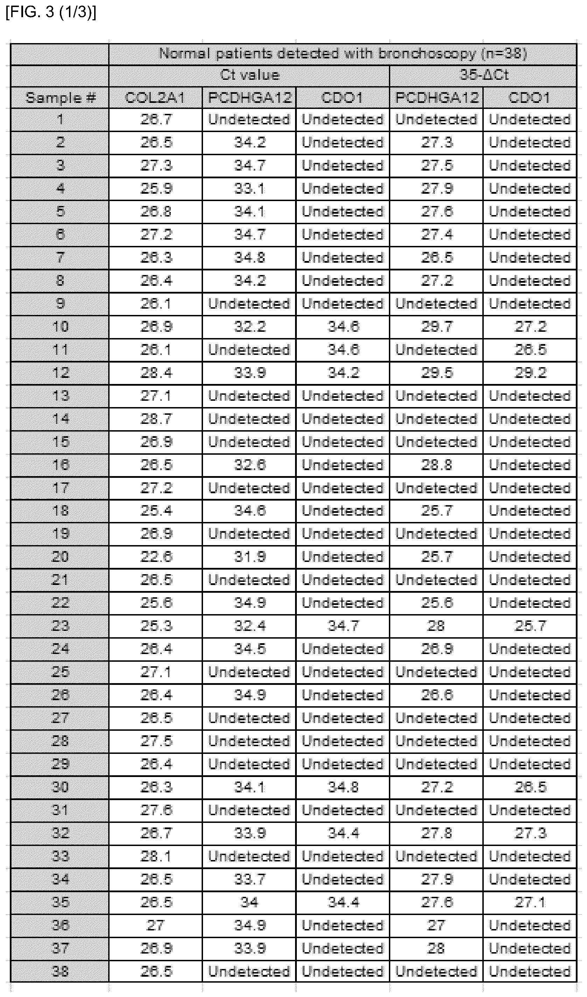

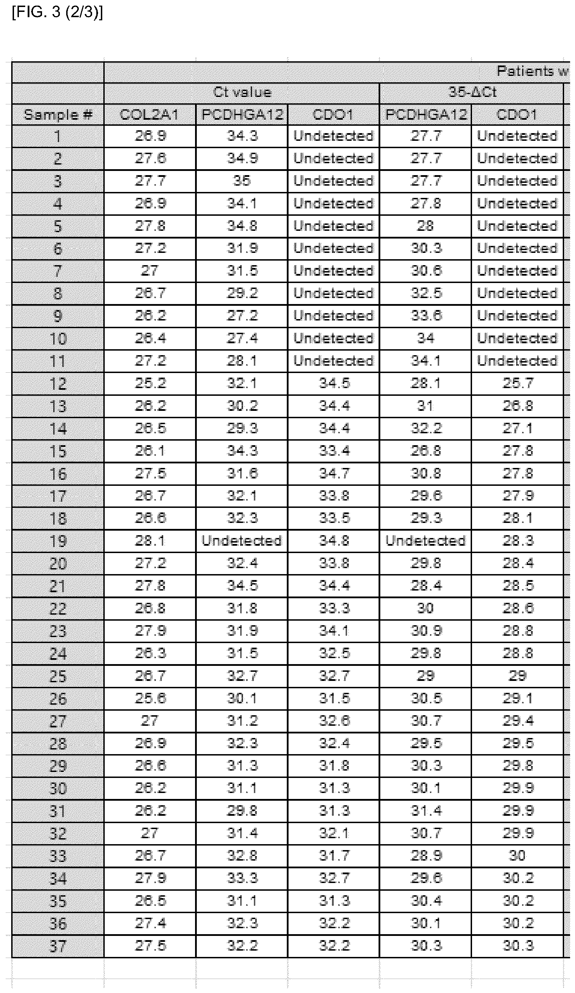

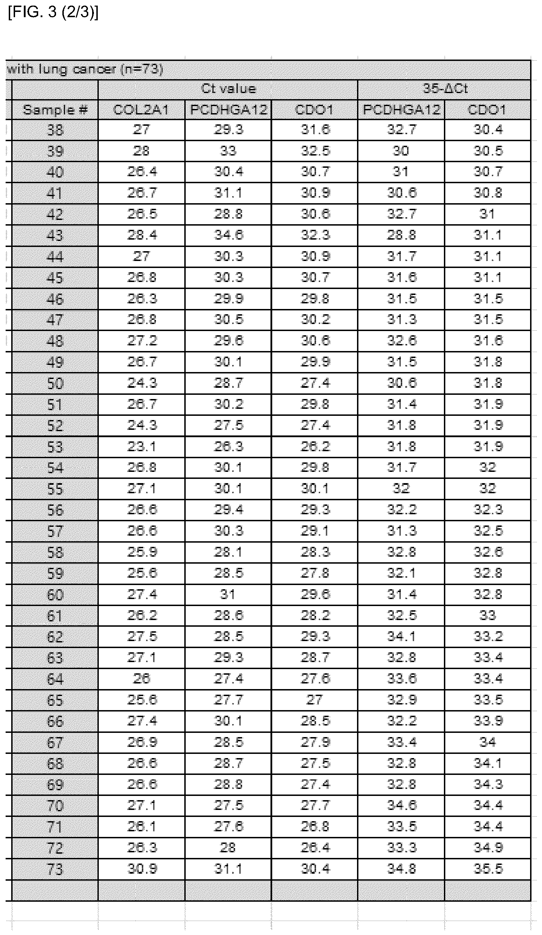

FIG. 3 shows the results of diagnosing lung cancer by detecting methylation of PCDHGA12 and CDO1 genes in bronchial lavage fluid from patients with benign lung disease and lung cancer.

### Detailed Description of the Invention and Specific Examples

Unless otherwise defined, all technical and scientific terms used herein shall have the same meaning as commonly understood by those skilled in the art. In general, the nomenclature used herein is well known and in common use in the art.

The present invention relates to a method for detecting methylation of a lung cancer-specific methylated gene, CDO1, for detecting lung cancer, and more specifically, provides information for diagnosing lung cancer by measuring methylation of a specifically methylated gene in lung cancer cells. Furthermore, the present invention provides improved lung cancer detection capability when combined with methylation of PCDHGA12, a lung cancer-specific methylation gene.

The inventors of the present invention have found that by using a primer that specifically amplifies a plurality of methylated lung cancer marker genes, the sensitivity and specificity of methylated DNA detection can be improved compared to methylation detection based on a single lung cancer marker gene. In a specific example according to the present invention, by detecting methylation of the PCDHGA12 (Protocadherin Gamma Subfamily A, 12) gene and CDO1 (Cysteine Dioxygenase Type 1) gene, the sensitivity and specificity for lung cancer diagnosis were improved, confirming its usefulness for lung cancer diagnosis, compared to methylation detection of the SDC2 gene.

To achieve the above objectives, the present invention relates to a method for providing information for lung cancer diagnosis, comprising the following steps: (a) treating a sample with one or more reagents that differentially modify the methylated PCDHGA12 (Protocadherin Gamma Subfamily A, 12) gene and the CDO1 (Cysteine Dioxygenase Type 1) gene, the unmethylated PCDHGA12 gene and the CDO1 gene; and (b) treating a primer that specifically amplifies the methylated PCDHGA12 gene and the CDO1 gene.

Accordingly, the present invention relates to a method for diagnosing lung cancer, comprising the following steps: (a) treating a sample with one or more reagents that differentially modify the methylated PCDHGA12 (Protocadherin Gamma Subfamily A, 12) gene and the CDO1 (Cysteine Dioxygenase Type 1) gene, the unmethylated PCDHGA12 gene and the CDO1 gene; and (b) treating a primer that specifically amplifies the methylated PCDHGA12 gene and the CDO1 gene.

In the present invention, step (a) is treating the sample with one or more reagents that differentially modify the methylated PCDHGA12 gene and the CDO1 gene, the unmethylated PCDHGA12 gene and the CDO1 gene.

As used herein, "methylation" refers to the attachment of a methyl group to the fifth carbon of the cytocine base ring to form 5-methylcytosine (5-mC), wherein 5-methylcytosine is always at the C of the CG dinucleotide only (5'-mCG-3'), and such CG is commonly referred to as CpG. Such methylation of CpG inhibits the expression of repetitive sequence in the genome, such as alu and transposon, and is the most common site of extragenomic changes in mammalian cells. The 5-mC of this CpG is spontaneously deaminated and changed to T, resulting in CpG in the mammalian genome appearing only 1% of the time, far less than it should normally (1/4 x 1/4 = 6.25%).

Some CpGs appear exceptionally densely clustered, and these are called CpG sites (CpG islands). CpG islands are defined as regions that are 0.2-3 kb in length, have C and G base distribution percentage of more than 50%, and a high concentration of CpGs with a distribution percentage of at least 3.75%. There are about 45,000 CpG islands in the entire human genome, and they are particularly concentrated in promoter regions that regulate gene expression. In fact, CpG islands appear in the promoters of housekeeping genes, which account for about half of all human genes.

Nucleic acids isolated from a specimen are obtained from a biological sample of the specimen. To diagnose lung cancer or advanced stages of lung cancer, nucleic acids must be isolated from lung tissue by scrapings or biopsies. Such samples can be obtained by various medical procedures known in the art.

The degree of methylation of the nucleic acids of the sample obtained from the specimen is measured by comparison with the same nucleic acid portion of a specimen having no cell growth abnormalities of lung tissue. Hypermethylation means the presence of methylated alleles in one or more nucleic acids. Specimen having no cell growth abnormalities in lung tissue does not show methylated alleles when the same nucleic acids are tested.

"Normal" cells are cells that do not exhibit abnormal cell morphology or changes in cytologic properties. "Tumor" cells are cancer cells, and "non-neoplastic" cells are cells that are part of the diseased tissue, but are determined not to be tumor sites.

According to the present invention, the methylation stage of one or more nucleic acids isolated from a specimen can be determined for early diagnosis of cell growth abnormalities in lung tissue of the specimen. The methylation stage of the one or more nucleic acids can be compared to the methylation state of one or more nucleic acids isolated from a sample that does not have cell growth abnormalities of lung tissue. The nucleic acid is preferably a CpG-containing nucleic acid, such as a CpG island.

According to the present invention, the methylation stage of one or more nucleic acids isolated from a specimen can be determined for diagnosis of cell growth abnormalities in lung tissue of the specimen. The methylation stage of the one or more nucleic acids can be compared to the methylation state of one or more nucleic acids isolated from a sample that does not have cell growth abnormalities of lung tissue.

"Predisposition" means a predisposition to the above cell growth abnormalities. A predisposed specimen is a specimen that does not yet have cell growth abnormalities, but has an increased tendency to have or develop cell growth abnormalities.

The presence of CpG methylation in the target DNA may be an indicator of disease, for example, by measuring CpG methylation at any one of a promoter, a 5' untranslated region, and an intron of the target DNA.

The CpG-containing gene is typically DNA. However, the method of the present invention can be applied, for example, to samples containing DNA or RNA comprising DNA and mRNA, wherein the DNA or RNA can be single-stranded or double-stranded, or to samples containing DNA-RNA hybrids.

Mixtures of nucleic acids may also be used. As used in the present invention, "multiple" includes both multiple specific nucleic acid sequence sites to be detected within a single gene and multiple target DNAs in one tube (single reactor). The specific nucleic acid sequence to be detected may be a fraction of a larger molecule, or it may be present as an isolated molecule in which the specific sequence constitutes the entire nucleic acid sequence from the beginning. The nucleic acid sequence need not be a nucleic acid present in pure form, and the nucleic acid may be a small fraction within a complex mixture, such as one containing the entire human DNA.

Specifically, the present invention is directed to detecting methylation of a plurality of target DNAs in a sample in a single reactor, wherein the sample may comprise a plurality of multiple target DNAs, wherein the target DNAs can be any gene that affects the development or progression of lung cancer without any limitation when it is aberrantly methylated and the expression thereof is inhibited.

In the present invention, the sample can be derived from a human body, for example, the sample can be lung cancer tissue, cells, feces, urine, blood, serum or plasma.

The one or more reagents that differentially modify the methylated and unmethylated DNA can be used without limitation as long as they are capable of distinguishing between unmethylated and methylated cytosine bases, such as, but not limited to, bisulfite, hydrogensulfite, disulfite, and combinations thereof. Specifically, methylated cytosine bases are not converted by the above reagents, while unmethylated cytosine bases can be converted to bases other than uracil or cytosine.

In the present invention, step (b) is the step of processing a primer that specifically amplifies the methylated PCDHGA12 gene and CDO1 gene.

The primer can be used simultaneously, for example, by pairing forward and reverse primers for PCR. Furthermore, the primers are designed so that the methylated "C" of the CpG site is necessarily located at the 3' end of the primers, which makes them highly specific for methylation detection. The forward primer that specifically amplifies the methylated PCDHGA12 gene may comprise, for example, the sequence of SEQ ID NO: 1. The reverse primer may comprise, for example, the sequence of SEQ ID NO: 2. The primer that specifically amplifies the methylated PCDHGA12 gene may comprise the primer pair of SEQ ID Nos: 1 and 2.

The forward primer that specifically amplifies the methylated CDO1 gene may comprise, for example, the sequence of SEQ ID NO: 4. The reverse primer may comprise, for example, the sequence of SEQ ID NO: 5. The primer that specifically amplifies the methylated CDO1 gene may comprise the primer pair of SEQ ID Nos: 4 and 5.

The present invention may further comprise the step of processing a probe capable of complementarily hybridizing to the methylated PCDHGA12 gene and the methylated CDO1 gene, respectively, specifically amplified by the primer in step (b) above.

In a hybridization reaction, the conditions used to achieve a strict specific level vary depending on the nature of the hybridized nucleic acids. For example, the length of the nucleic acid sites being hybridized, the degree of homology, the nucleotide sequence composition (e.g., GC/AT ratio), and the type of nucleic acid (e.g., RNA, DNA) are considered in selecting hybridization conditions. An additional consideration is whether the nucleic acid is immobilized, e.g., on a filter.

Examples of very stringent conditions include 2X SSC/0.1% SDS at room temperature (hybridization condition); 0.2X SSC/0.1% SDS at room temperature (low stringency condition); 0.2X SSC/0.1% SDS at 42°C (moderate stringency condition); 0.1X SSC at 68°C (high stringency condition). The washing process may be performed using any one of these conditions, e.g., high stringency condition, or each of the above conditions, and may be repeated in the order described above for 10 to 15 minutes each, in whole or in part. However, as described above, optimal conditions vary depending on the particular hybridization reaction involved therein and can be determined by experimentation. In general, high stringency conditions are used for hybridization of important probes.

A probe capable of hybridizing complementarily to the amplified methylated CDO1 gene or methylated PCDHGA12 gene may comprise, for example, a sequence of SEQ ID NO: 3 or 6. A probe capable of hybridizing complementarily to the amplified methylated CDO1 gene may comprise, for example, a sequence of SEQ ID NO: 3. A probe capable of hybridizing complementarily to the amplified methylated *PCDHGA12* gene may comprise, for example, a sequence of SEQ ID NO: 6.

In some cases, the probe is labeled for detection, and may be labeled, for example, with a radioisotope, a fluorescent compound, a bioluminescent compound, a chemiluminescent compound, a metal chelator, or an enzyme. Suitable labeling of such probe is well known in the art and can be accomplished by conventional methods.

The amount of the amplification product can be detected by a fluorescent signal. Intercalating methods, which use a reagent called an intercalator that binds to the double-stranded DNA of the amplification product to which the probe is bound and fluoresces, or oligonucleotides labeled with a fluorophore at the 5' end and a quencher at the 3' end, are used.

Amplification according to the present invention can be performed by real-time quantitative amplification, for example by real-time polymerase chain reaction (Real-Time PCR), wherein the amount of PCR amplification product can be detected by a fluorescent signal. As the real-time polymerase chain reaction proceeds, the intensity of the fluorescence signal increases with increasing amounts of polynucleotide, resulting in an amplification profile curve that exhibits the intensity of the fluorescence signal depending on the number of amplification cycles.

The amplification profile curve is typically divided into a baseline region, which shows a background fluorescent signal that does not reflect the actual amount of polynucleotide, an exponential region, which shows an increase in fluorescent signal as the amount of polynucleotide product increases, and a plateau region, which shows no increase in fluorescent signal intensity as the PCR reaction reaches saturation.

In general, the point at which the baseline region transitions to the exponential region, i.e., the fluorescent signal intensity at which the amount of PCR amplification product reaches a fluorescently detectable amount, is called the threshold, and the number of amplification cycles corresponding to the threshold in the amplification profile curve is called the threshold cycle (Ct) value.

By measuring the Ct value and analyzing a standard curve in which the concentration is determined based on the Ct (threshold cycle) value for the standard substance, the concentration of the amplified gene can be determined, thereby determining the methylation-specific sensitivity and/or specificity.

In one example, the methylation detection can be performed by a method selected from the group consisting of PCR, methylation specific PCR, real time methylation specific PCR, PCR using a methylation DNA specific binding protein, PCR using a methylation DNA specific binding antibody, quantitative PCR, gene chip, sequencing, sequencing by synthase, and sequencing by ligation.
(1) Methylation specific PCR (methylation specific PCR): When detected by the above methylation specific PCR, bisulfite treatment causes the cytosine at the 5'-CpG'-3 site to remain as cytosine when methylated, and to be converted to uracil when unmethylated. Therefore, a primer corresponding to the site where the 5'-CpG-3' sequence is present can be prepared for the sequence converted after bisulfite treatment. When PCR is performed using the primer, a PCR product is produced from the primer corresponding to the methylated sequence if it is methylated, and the methylation can be confirmed by agarose gel electrophoresis. The methylation detection probe may be, but is not limited to, a TaqMan, a Molecular Beacon, a self-reporting probe, or a probe having an energy transfer labeling function.
(2) Real time methylation specific PCR: The real time methylation specific PCR is a conversion of the methylation specific PCR method into a real-time measurement method, which is to treat the geomic DNA with bisulfite, design a PCR primer corresponding to the methylated case, and perform real time PCR using this primer. At this time, there are two detection methods: detection using TaqMan probes complementary to the amplified sequence and detection using Sybergreen. Therefore, real time methylation specific PCR can selectively quantify only methylated DNA. In this case, a standard curve can be constructed using in vitro methylated DNA samples, and a gene without a 5'-CpG-3' sequence in the sequence can be amplified as a negative control for standardization, and the degree of methylation can be quantitatively analyzed.
(3) PCR or quantitative PCR or DNA chip using a methylation DNA specific binding protein: The PCR or DNA chip method using a methylation DNA specific binding protein DNA specific binding protein can selectively isolate only methylated DNA because the protein binds specifically to methylated DNA when mixed with DNA.
   Methylation can also be measured by quantitative PCR, in which methylation DNA isolated with a methylation DNA specific binding protein DNA specific binding protein is labeled with a fluorescent dye and hybridized to a DNA chip with complementary probes.
(4) Detection of differential methylation - bisulfite sequencing method: Another method for detecting a nucleic acid containing a methylated CpG comprises the step of contacting a sample containing the nucleic acid with an agent that modifies unmethylated cytosine and the step of amplifying the CpG-containing nucleic acid in the sample using a CpG-specific oligonucleotide primer. Here, the oligonucleotide primer may discriminate between modified methylated and unmethylated nucleic acids to detect methylated nucleic acids. The amplification step is optional, preferably but not essential. The method relies on a PCR reaction that distinguishes between modified (e.g., chemically modified) methylated and unmethylated DNA.
(5) Bisulfite sequencing method: Another method for detecting a nucleic acid containing a methylated CpG comprises the step of contacting a sample containing the nucleic acid with an agent that modifies unmethylated cytosine and amplifying the CpG-containing nucleic acid in the sample using a methylation-independent oligonucleotide primer. Here, the oligonucleotide primer may not discriminate between modified methylated and unmethylated nucleic acids to amplify methylated nucleic acids. The amplified product may be sequenced by the Sanger method using a sequencing primer, or by next generation sequencing method, with reference to bisulfite sequencing for detection of methylated nucleic acids.
(6) The next generation sequencing method can be conducted by sequencing by synthesis or sequencing by ligation. This method spatially separates single DNA fragments and amplifies in situ (clonal amplification), and sequences, instead of creating bacterial clones. This is sometimes referred to as massively parallel sequencing because hundreds of thousands of fragments are read simultaneously.

Basically, it is a sequencing by synthesis method, where mono- or dinucleotides are sequentially spliced together to obtain a signal, such as pyrosequencing, ion torrent, and Solexa.

NGS instruments based on sequencing by synthesis include the 454 platform from Roche, the HiSeq platform from Illumina, the Ion PGM platform from Life Technology, and finally the PacBio platform from Pacific BioSciences. The 454 and Ion PGM use emersion PCR as a clonal amplification method, while HiSeq uses bridge amplification. Sequencing by synthesis method reads sequences by detecting phosphate, hydrogen ions, or pre-added fluorescence generated when DNA is synthesized by sequentially attaching one nucleotide after another. For sequence detection, the 454 uses a pyroseqeuncing method that utilizes phosphate, while the Ion PGM uses hydrogen ion detection. HiSeq and PacBio detect fluorescence to decode sequences.

Sequencing by ligation is a sequencing technique that utilizes DNA ligases to identify nucleotides at specific positions in a DNA sequence. Unlike most sequencing technologies that use polymerases, this technique does not use polymerases and uses the feature that DNA ligases do not ligate mismatched sequences. The SOLiD system is an example of this. In this technique, bases are read two at a time with a gap, and repeated five times independently with primer resets, so that each base is finally read twice in duplicate to increase accuracy.

In the case of sequencing by ligation, the dinucleotide primers corresponding to a given sequence, among a set of 16 combinations of dinucleotide primers, are sequentially ligated, and the combination of these ligations is finally analyzed to complete the sequence of the DNA.

Here, the next generation sequencing method can be conducted by sequencing by synthesis or sequencing by ligation. The methylation DNA specific binding protein is not limited to MBD2bt, and the antibody is not limited to a 5'-methylcytosine antibody.

With respect to the primer used in the present invention, treatment of the reagent, e.g., bisulfite, according to step (a) above causes the cytosine at the 5'-CpG'-3 site to remain cytosine if methylated and to be converted to uracil if unmethylated. Therefore, a primer corresponding to the site where the 5'-CpG-3' sequence is present can be prepared for the sequence converted after treatment with a reagent, e.g., bisulfite.

The primer can be made to be "substantially" complementary to each strand of the gene locus to be amplified. This means that the primer has sufficient complementarity to hybridize to the corresponding nucleic acid strands under the conditions of the polymerization reaction.

In another aspects, the present invention also provides a composition for diagnosing lung cancer, comprising (a) one or more reagents that differentially modify the methylated PCDHGA12 gene and the CDO1 gene, the unmethylated PCDHGA12 gene and the CDO1 gene; and (b) a primer that specifically amplifies the methylated PCDHGA12 gene and the methylated CDO1 gene.

The constitutions included in the composition according to the present invention overlap with the previously described constitutions, and therefore the description of them applies equally.

In another aspect, the present invention relates to a kit for detecting methylation of target DNA comprising the composition.

In one example, the kit may comprise a compartmentalized carrier means for holding a sample, a container containing reagents, and a container containing primers for each of the methylated PCDHGA12 gene and the methylated CDO1 gene. In some cases, the kit may further comprise a container containing a probe for detecting each of the methylated PCDHGA12 gene and methylated CDO1 gene amplification products.

The carrier means is suitable for containing one or more containers, such as bottles or tubes, each containing independent components used in the method of the present invention. From the specification of the present invention, a person having ordinary skill in the art can easily dispense the required formulation from the container.

The present invention will now be described in more detail with reference to the following examples. These examples are intended solely to illustrate the present invention, and it will be apparent to one of ordinary skill in the art that the scope of the present invention is not to be construed as limited by these examples.

### Example 1. Validation of PCDHGA12 and CDO1 methylation gene detection primers and probes

Primer sets for multiplex polymerase chain reaction (multiplex PCR) for simultaneous detection of methylation were constructed for the PCDHGA12 and CDO1 genes. Methylation-specific oligonucleotide primer and probe sets were designed using MethPrimer (http://www.urogene.org/cgi-bin/methprimer/methprimer.cgi) for the PCDHGA12 and CDO1 gene sequences converted with bisulfite (Table 1). The PCDHGA12 gene was designed based on the exon 1 region sequence, and the CDO1 gene was designed based on the promoter region sequence. Oligonucleotide primers and probes specific for the control gene COL2A1 were also designed (Table 1).

**Table 1. Sequence of PCDHGA12 and CDO1 methylation gene amplification primers and probes**

| Gene name | Primer or probe | Sequence (5' → 3' ) | SEQ ID No. |
|---|---|---|---|
| *PCDHGAI 2* | Forward | CGGTACGTATAGGTATCGC | 1 |
| | Reverse | CCAAAACCAAATTCTCCGAAACGCTCGCG | 2 |
| | Probes | [HEX]CGTATTCGCGTGATGGTTTTGGATGC[IABkFQ] | 3 |
| *CDO1* | Forward | GAGAGATTGCGCGGAGTTTAC | 4 |
| | Reverse | CGAAAACGAAAAAACCCTACGAACACGACTC | 5 |
| | Probe | [FAM]TTTTTGGGAAGGCGCGGAGTTCGGGGAAGT[IA BkFQ] | 6 |
| *COL2A1* | Forward | TAGGAGTATTAGTAATGTTAGGAGTA | 7 |
| | Reverse | CTACCCCAAAAAAACCCAATCC | 8 |
| | Probe | [Cy5]AGAAGAAGGGAGGGGTGTTAGGAGAGG[IABkF Q] | 9 |

In order to confirm the methylation-specific detection of the designed primers and probes for multiplex PCR of PCDHGA12 and CDO1 genes, quantitative methylation-specific real time polymerase chain reaction (qMSP) method was used using methylated and unmethylated control DNA (EpiTect PCR control DNA set (Qiagen, cat. no. 59695)). The COL2A1 gene was used as an internal control gene.

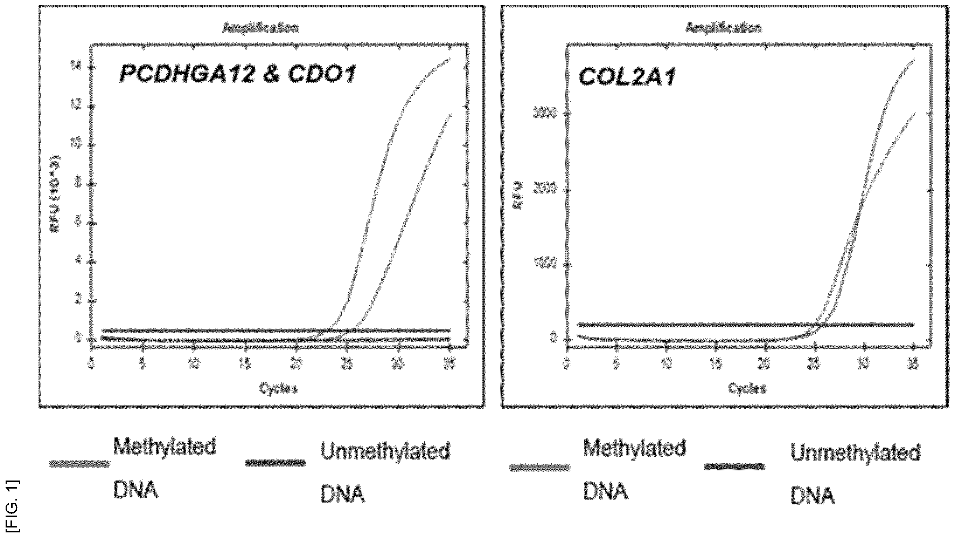

A total of 25 µl PCR reaction solution (bisulfite-converted template human DNA, 20 ng/5 µl; Qplex Master Mix (CellSafe, Korea), 10 µl; methylation-specific PCR primers (IDT, USA), 1 µl each (1 to 10 pmole/µl); methylation-specific TaqMan probe (IDT, USA), 1 µl each (2.5 to 5 pmole/µl); D.W. 1 µl) were prepared and PCR reacted with template human DNA converted with bisulfite (Table 2). The PCR conditions were conducted at 95°C for 5 minutes, followed by 5 cycles of 95°C for 15 seconds and 72°C for 45 seconds, and qMSP step was conducted at 95°C for 15 seconds and 58°C for 45 seconds for a total of 35 cycles, and the amplification of the PCR product of qMSP was checked in real time using a CFX96 (Bio-Rad) instrument (Fig. 1).

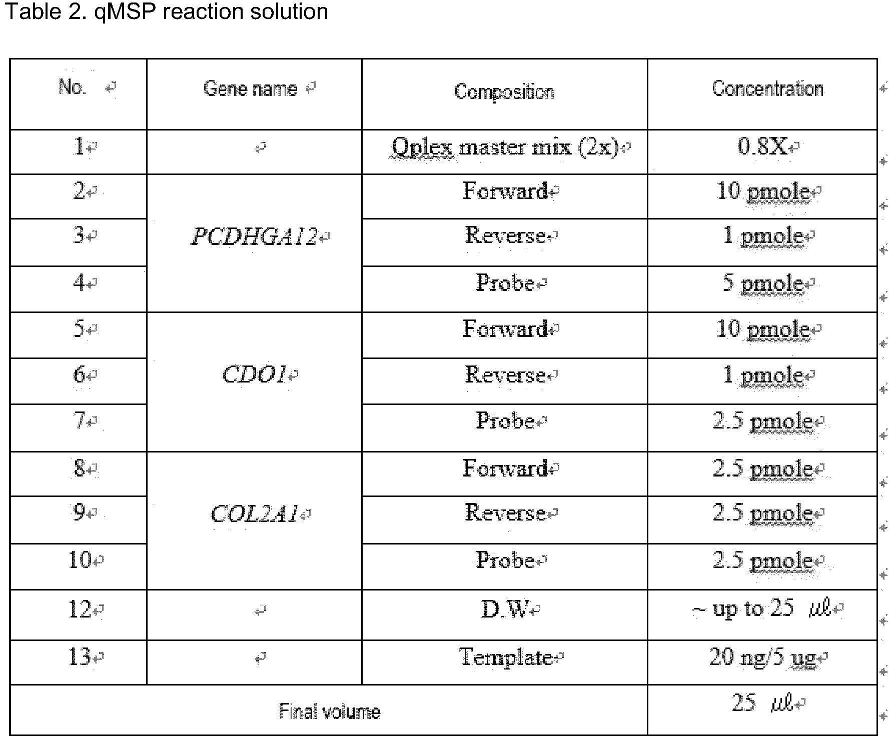

As shown in FIG. 1, the PCDHGA12 and CDO1 genes and the control gene COL2A1 were amplified together in methylated DNA, whereas in unmethylated DNA, the PCDHGA12 and CDO1 genes were not amplified, but only the control gene COL2A1 was amplified, confirming that the methylation-specific primers for the PCDHGA12 and CDO1 genes work properly.

### Example 2. Confirmation of increased lung cancer detection performance by combination of CDO1 and PCDHGA12 gene methylation in bronchial lavage fluid DNA

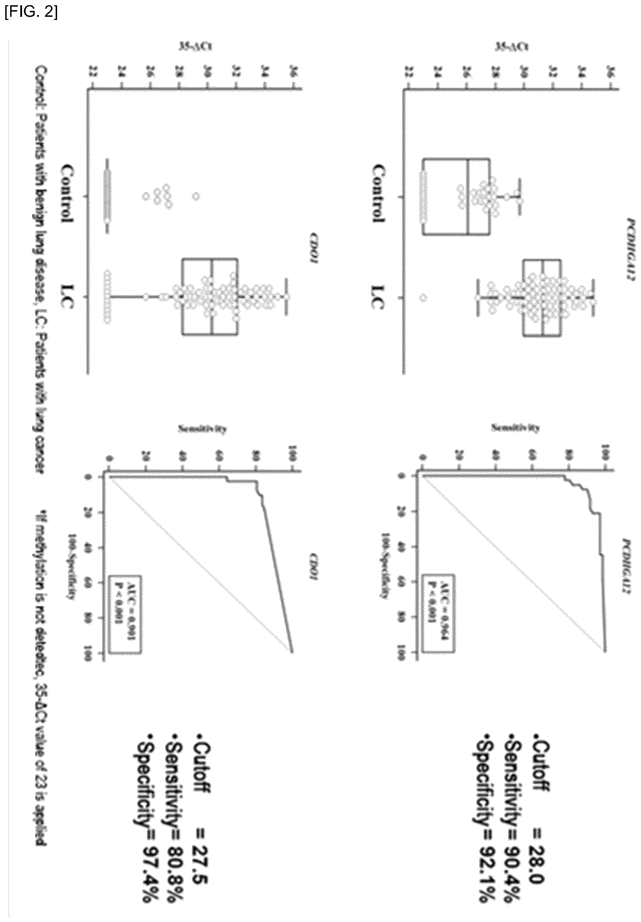

To determine whether the combination of CDO1 gene and the methylation of the PCDHGA12 gene (Korean Patent No. 10-1106727), an existing methylation biomarker for early lung cancer diagnosis (Table 3), improves the detection of lung cancer, the methylation of the PCDHGA12 and CDO1 genes in bronchial lavage fluid DNA collected through bronchoscopy from 111 patients, including 38 patients with benign lung disease (Kunyang University Hospital) and 73 patients with lung cancer (Kunyang University Hospital) (FIG. 2). The methylation of PCDHGA12 and CDO1 genes in these samples was measured using the qMSP method described in Example 2.

PCR equipment was CFX96 (Bio-Rad), and bisulfite conversion was performed using the EZ Methylation Gold Kit (Catalog No. D5006, Zymo Research, USA).

The methylation level of each gene was expressed by the 35-dCt (PCDHGA12 or CDO1 Ct - COL2A1 Ct) (Chung W et al., Cancer Epidemiol Biomarkers Prev., 2011;20(7):1483-1491) method. A higher 35-dCt value indicates a higher level of methylation. To evaluate the performance of each gene in the diagnosis of lung cancer, Receiver Operating Characteristic (ROC) analysis was performed using the MedCalc program (MedCalc software, Ballgier) to establish the optimal cutoff for a positive result, and calculate sensitivity and specificity.

When methylation of the CDO1 gene was measured using bronchial lavage fluid DNA treated and converted with bisulfite, the methylation-positive cutoff was calculated to be 27.5, and only 1 out of 38 patients with benign lung disease was methylation-positive, with a specificity of 97.4%; in lung cancer patients, 59 out of 73 patients were methylation-positive, with a sensitivity of 80.8%. When methylation of the PCDHGA12 gene was combined, 3 out of 38 patients with benign lung disease were methylation-positive, with a specificity of 92.1%, which indicates no significant decrease (P = 0.615, Fisher's exact test). In terms of sensitivity, 68 out of 73 lung cancer patients were methylation-positive, with additional 9 lung cancer patients being positive compared to CDO1 alone, for a total of 68 (68/73), which indicated significantly increased sensitivity to 93.2% (P < 0.047, Fisher's exact test) (Table 3). Thus, the combination of CDO1 gene methylation and PCDHGA12 gene methylation significantly increased the ability to detect lung cancer.

**Table 3. Sensitivity and specificity of detection of PCDHGA12 and CDO1 methylation combination**

| Number of samples | Gene name | Cutoff (35- ΔCt) | Methylation | |
|---|---|---|---|---|
| | | | Sensitivity | Specificity |
| Overall (n = 111) | *PCDHGA12* | >28.0 | 90.4 % (66/73) | 92.1 % (3/38) |
| | *CDO1* | >27.5 | 80.8 % (59/73) | 97.4 % (1/38) |
| | *PCDHGA12 + CDO1* | - | 93.2 % (68/73) | 92.1 % (3/38) |

### Industrial Availability

The present invention is useful because, by detecting methylation with high detection sensitivity using a primer that specifically amplifies a plurality of methylated lung cancer marker genes, the detection of lung cancer can be improved compared to the detection method using a single marker gene, enabling accurate and rapid lung cancer diagnosis.

While the foregoing has described in detail certain aspects of the present invention, it will be apparent to one of ordinary skill in the art that these specific descriptions are merely preferred examples and are not intended to limit the scope of the present invention. Accordingly, the substantial scope of the present invention will be defined by the appended claims and their equivalents.

## Claims

1. A method for providing information for lung cancer diagnosis, comprising the following steps:
(a) treating a sample with one or more reagents that differentially modify the methylated PCDHGA12 (Protocadherin Gamma Subfamily A, 12) gene and the CDO1 (Cysteine Dioxygenase Type 1) gene, the unmethylated PCDHGA12 gene and the CDO1 gene; and (b) treating primers that specifically amplify the methylated PCDHGA12 gene and the CDO1 gene.

2. The method according to claim 1, wherein the reagent is bisulfite, hydrogensulfite, disulfite, or a combination thereof.

3. The method according to claim 1, wherein the reagent treatment converts the at least one cytosine base to a base different from uracil or cytosine.

4. The method according to claim 1, wherein the primer that specifically amplifies the methylated CDO1 gene may comprise the primer pair of SEQ ID Nos: 4 and 5.

5. The method according to claim 1, wherein the primer that specifically amplifies the methylated PCDHGA12 gene may comprise the primer pair of SEQ ID Nos: 1 and 2.

6. The method according to claim 1, wherein the method may further comprise the step (c) of processing probes capable of complementarily hybridizing to the methylated PCDHGA12 gene and the methylated CDO1 gene, respectively, specifically amplified by the primer in step (b) above.

7. The method according to claim 6, wherein the probe capable of hybridizing complementarily to the amplified methylated CDO1 gene may comprise, for example, a sequence of SEQ ID NO: 6.

8. The method according to claim 6, wherein the probe capable of hybridizing complementarily to the amplified methylated PCDHGA12 gene may comprise, for example, a sequence of SEQ ID NO: 3.

9. The method according to claim 1, wherein the methylation detection can be performed by a method selected from the group consisting of PCR, methylation specific PCR, real time methylation specific PCR, PCR using a methylation DNA specific binding protein, PCR using a methylation DNA specific binding antibody, quantitative PCR, gene chip, sequencing, sequencing by synthase, and sequencing by ligation.

10. The method according to claim 6, wherein the methylation is detected by detecting a substance that binds to the probe and exhibits fluorescence.

11. A composition for diagnosing lung cancer, comprising one or more reagents that differentially modify the methylated PCDHGA12 (Protocadherin Gamma Subfamily A, 12) gene and the CDO1 (Cysteine Dioxygenase Type 1) gene, the unmethylated PCDHGA12 gene and the CDO1 gene; and primers that specifically amplify the methylated PCDHGA12 gene and the CDO1 gene.

12. The composition according to claim 11, wherein the reagent is bisulfite, hydrogensulfite, disulfite, or a combination thereof.

13. The composition according to claim 11, wherein the reagent treatment converts the at least one cytosine base to a base different from uracil or cytosine.

14. The composition according to claim 11, wherein the primer that specifically amplifies the methylated CDO1 gene may comprise the primer pair of SEQ ID Nos: 4 and 5.

15. The composition according to claim 11, wherein the primer that specifically amplifies the methylated PCDHGA12 gene may comprise the primer pair of SEQ ID Nos: 1 and 2.

16. The composition according to claim 11, which further comprises the step (c) of processing probes capable of complementarily hybridizing to the methylated PCDHGA12 gene and the methylated CDO1 gene, respectively, specifically amplified by the primer.

17. The composition according to claim 16, wherein the probe capable of hybridizing complementarily to the amplified methylated CDO1 gene may comprise a sequence of SEQ ID NO: 6.

18. The composition according to claim 16, wherein the probe capable of hybridizing complementarily to the amplified methylated PCDHGA12 gene may comprise a sequence of SEQ ID NO: 3.

19. A kit for diagnosing lung cancer comprising the composition according to any one of claims 11 to 18.
